Europäisches Patentamt

European Patent Office

Office européen des brevets

(11) Publication number: **0 273 603 B1**

# EUROPEAN PATENT SPECIFICATION

(45) Date of publication of patent specification: **11.03.92** (51) Int. Cl.5: **A61K 31/71, A61K 9/08**

(21) Application number: **87310632.2**

(22) Date of filing: **03.12.87**

Divisional application 91102986.6 filed on 03/12/87.

(54) **Injectable ready-to-use solutions containing an antitumor anthracycline glycoside.**

(30) Priority: **05.12.86 GB 8629193**
**22.06.87 US 64653**

(43) Date of publication of application:
**06.07.88 Bulletin 88/27**

(45) Publication of the grant of the patent:
**11.03.92 Bulletin 92/11**

(84) Designated Contracting States:
**AT BE CH DE ES FR GB GR IT LI LU NL SE**

(56) References cited:
**FR-A- 2 405 957**
**GB-A- 2 178 311**

**CHEMICAL ABSTRACTS, vol. 104, 1986, page 439, abstract no. 155834q, Columbus, Ohio, US; J.H. BEIJNEN et al.: "Stability of anthracycline antitumor agents in infusion fluids"**

**MERCK INDEX, 10th edition, page 499, no. 3435 "Doxorubicin"**

**J. Parenter, SCI-TECHNOL., 1985, vol. 39, no. 6, pages 220-222**

(73) Proprietor: **FARMITALIA CARLO ERBA S.r.L.**
**Via Carlo Imbonati 24**
**I-20159 Milano(IT)**

(72) Inventor: **Gatti, Gaetano**
**viale Fulvio Testi 42**
**Sesto San Giovanni Milan(IT)**
Inventor: **Oldani, Diego**
**via San Giovanni 48**
**Robecco sul Naviglio Milan(IT)**
Inventor: **Bottoni, Giuseppe**
**via Corridoni 85**
**Bergamo(IT)**
Inventor: **Confalonieri, Carlo**
**via Ticino 5**
**Cusano Milanino Milan(IT)**
Inventor: **Gambini, Luciano**
**via Piave 16**
**Cornaredo Milan(IT)**
Inventor: **De Ponti, Roberto**
**via Degli Astri 22**
**Milan(IT)**

(74) Representative: **Woods, Geoffrey Corlett et al**
**J.A. KEMP & CO. 14 South Square Gray's Inn**
**London WC1R 5EU(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a storage stable, injectable ready-to-use solution of an antitumor anthracycline glycoside, e.g. doxorubicin, and to a process for preparing such a solution.

The anthracycline glycoside compounds are a well known class of compounds in the antineoplastic group of agents, of which doxorubicin is a typical, and the most widely used, representative: Doxorubicin. Anticancer Antibiotics, Federico Arcamone, 1981, Publ: Academic Press, New York, N.Y.; Adriamycin Review, EROTC International Symposium, Brussels, May, 1974, edited by M. Staquet, Publ. Eur. Press Medikon, Ghent, Belg.; and Results of Adriamycin Therapy, Adriamycin Symposium at Frankfurt/Main 1974 edited by M.Ghione, J.Fetzer and H.Maier, publ.: Springer, New York, N.Y.

At present, anthracycline glycoside antitumor drugs, in particular, e.g. doxorubicin, are solely available in the form of lyophilized preparations, which need to be reconstituted before administration. Both the manufacture and the reconstitution of such preparations expose the involved personnel (workers, pharmacists, medical personnel, nurses) to risks of contamination which are particularly serious due to the toxicity of the antitumor substances.

The Martindale Extra Pharmacopoeia 28th edition, page 175 left column, reports on adverse effects of antineoplastic drugs and recommends that "They must be handled with great care and contact with skin and eyes avoided; they should not be inhaled. Care must be taken to avoid extravasation since pain and tissue damage may ensue.". Similarly, Scand. J. Work Environ Health vol.10 (2), pages 71-74 (1984), as well as articles in Chemistry Industry, Issue July 4, 1983, page 488, and Drug-Topics-Medical-Economics-Co,Issue February 7, 1983, page 99 report about severe adverse effects observed in medical personnel exposed to use of cytostatic agents, including doxorubicin.

To administer a lyophilized preparation, double handling of the drug is required. The lyophilized cake first has to be reconstituted and then administered. Moreover, in some cases, the complete dissolution of the powder may require prolonged shaking because of solubilization problems. Reconstitution of a lyophilized cake or powder can result in formation of aerosol droplets which can be inhaled or can come into contact with skin or mucous membranes of those handling the solution.

As the risks connected with the manufacture and the reconstitution of a lyophilized preparate would be highly reduced if a ready-to-use solution of the drug were available, we have developed a stable, therapeutically acceptable intravenously injectable solution of an anthracycline glycoside drug, e.g. doxorubicin, whose preparation and administration does not require either lyophilization or reconstitution.

GB-A-2178311 describes and claims sterile, pyrogen-free, anthracycline glycoside solutions which consist essentially of a physiologically acceptable salt of an anthracycline glycoside such as doxorubicin dissolved in a physiologically acceptable solvent therefor, which have not been reconstituted from a lyophilizate and which have a pH of from 2.5 to 6.5. Especially preferred pH's are about 3 and about 5. The Examples illustrate solutions with pH's ranging from 2.62 to 3.14, with pH 4.6 and with pH 5.2. There is no mention of stabilising agents. Dextrose, lactose and mannitol are mentioned as tonicity adjustment agents but no indication is given of the proportions in which they may be used.

Beijnen et al, J. Parenter. Sci. Technol. 39, 6, 220-222, 1985 reports studies on the stability of doxorubicin, daunorubicin and four of their analogues in different infusion fluids. These fluids were 5% dextrose at pH 4.7, 3.3% dextrose + 0.3% NaCl at pH 4.4, Lactated Ringer's solution at pH 6.8 and 0.9% sodium chloride solution at pH 7.

According to the present invention, there is provided a ready-to-use, storage stable, sterile, pyrogen-free, injectable anthracycline glycoside solution which consists of a physiologically acceptable salt of an anthracycline glycoside dissolved in a physiologically acceptable aqueous solvent therefor at an anthracycline glycoside concentration of from 0.1 to 50 mg/ml, and which has not been reconstituted from a lyophilizate; characterised in that the pH of the solution has been adjusted to from 2.5 to 3.5 by means of a glycine buffer.

It is thus possible to provide solutions which are storage stable and have a commercially meaningful shelf-life.

Preferably the solution of the invention is provided in a sealed container, especially one made of glass. The solution can be provided in this way either in a unit dosage form or in a multiple dosage form.

Preferably the anthracycline glycoside is chosen from doxorubicin, 4'-epi-doxorubicin (i.e. epirubicin), 4'-desoxy-4'-iodo-doxorubicin, daunorubicin and 4-demethoxy-daunorubicin (i.e. idarubicin). Particularly preferred anthracycline glycosides are doxorubicin and 4'-epi-doxorubicin, especially doxorubicin.

Any physiologically acceptable salt of the anthracycline glycoside may be used for preparing the solution of the invention. Examples of suitable salts may be, for instance, the salts with mineral inorganic acids such as hydrochloric, hydrobromic, sulfuric or phosphoric acid and the salts with certain acids such as succinic, tartaric, ascorbic, citric, methanesulfonic or ethanesulfonic acid. The salt with hydrochloric acid is a particularly preferred salt, especially when the anthracycline glycoside is doxorubicin.

Any aqueous solvent which is physiologically acceptable and which is able to dissolve the anthracycline glycoside salt may be used. The solution of the invention may also contain one or more formulation adjuvants such as a co-solubilizing agent (which may be the same as a solvent), a tonicity adjustment agent, a preservative and a pharmaceutically acceptable chelating agent.

Suitable solvents and co-solubilizing agents may be, for instance, water e.g. Water for Injections; a 0.9% sodium chloride solution, i.e. physiological saline; and aqueous 5% dextrose solution; and mixtures of water and one or more of:

- an aliphatic amide, e.g. N,N-dimethylacetamide or N-hydroxy-2-ethyl-lactamide;
- an alcohol, e.g. ethanol or benzyl alcohol;
- a glycol or polyalcohol, e.g. propyleneglycol or glycerin;
- an ester of a polyalcohol, e.g. diacetine or triacetine;
- a polyglycol or polyether, e.g. polyethyleneglycol 400 or a propyleneglycol methylether;
- a dioxolane, e.g. isopropylidenglycerin;
- dimethylisosorbide; and
- a pyrrolidone derivative, e.g. 2-pyrrolidone, N-methyl-2-pyrrolidone or polyvinylpyrrolidone.

Examples of preferred solvents are water, physiological saline, an aqueous 5% dextrose solution, and mixtures of water and one or more of ethanol, polyethyleneglycol and dimethylacetamide.

Water, physiological saline and a 5% dextrose solution are particularly preferred.

Suitable tonicity adjustment agents may be, for instance, physiologically acceptable inorganic chlorides, e.g. sodium chloride, dextrose, lactose, mannitol or sorbitol

Preservatives suitable for physiological administration may be, for instance, esters of para-hydroxybenzoic acid (e.g., methyl, ethyl, propyl and butyl esters, or mixtures of them) and chlorocresol.

A suitable pharmaceutically acceptable chelating agent may be ethylenediaminotetraacetic acid (EDTA). The chelating agent is included in a minor amount, typically from 0.001 to 0.05 % by weight.

The above mentioned solvents, tonicity adjustment agents, preservatives and chelating agents can be used alone or as a mixture of two or more of them.

To adjust the pH within the range of from 2.5 to 3.5 a glycine buffer is added as desired.

The range of pH for the ready-to-use solution of the invention is from 2.5, e.g. from 2.6, to 3.5. A more preferred pH range is from 3 to 3.5. A pH of about 3 is particularly preferred especially where the solution of the invention contains sorbitol, dextrose, lactose or mannitol. Other preferred pH ranges are from greater than 3.14, e.g. from 3.2, to 3.5. A useful solution with a pH of from 2.62 to 3.14 further comprises a pharmaceutically acceptable chelating agent.

In the solutions of the invention the concentration of the anthracycline glycoside may vary within broad ranges, preferably from 1 mg/ml to 20

The preferred ranges of concentration may be slightly different for different anthracycline glycosides. Thus, for example, preferred concentrations for doxorubicin are from 2 mg/ml to 50 mg/ml, preferably from 2 mg/ml to 20 mg/ml, particularly appropriate values being 2 mg/ml and 5 mg/ml. Similar concentrations are preferred also for 4'-epi-doxorubicin and 4'-desoxy-4'-iodo-doxorubicin. Preferred ranges of concentration for daunorubicin and 4-demethoxy-daunorubicin are from 1 mg/ml to 20 mg/ml, concentrations of 1 mg/ml and 5 mg/ml being particularly appropriate.

Suitable packaging for the anthracycline glycoside solutions may be all approved containers intended for parenteral use, such as plastic and glass containers and ready-to-use syringes. Preferably the container is a sealed glass container, e.g. a vial or an ampoule. A hermetically sealed glass vial is particularly preferred.

The invention also provides a process for producing a storage stable, sterile, pyrogen-free, injectable, anthracycline glycoside solution, which process comprises dissolving a physiologically acceptable salt of an anthracycline glycoside, which salt is not in the form of a lyophilizate, in a physiologically acceptable aqueous solvent therefor at an anthracycline glycoside concentration of from 0.1 to 50mg/ml and the process being effected in such a manner that the resultant solution is sterile and pyrogen-free, characterised in that a glycine buffer is added to adjust the pH of the solution from 2.5 to 3.5 as desired. The resultant solution is then typically introduced into a container and sealed.

A suitable process comprises:

(i) dissolving the physiologically acceptable salt in the physiologically acceptable aqueous solvent;
(ii) adding the one or more formulation adjuvants selected from co-solubilizing agents, tonicity adjustment agents, preservatives and pharmaceutically acceptable chelating agents; and
(iii) adding the glycine buffer.

Any suitable procedure may be adopted to ensure that the resultant solution is sterile and pyrogen-free. Preferably, the solution is passed through a sterilising filter after addition of the glycine buffer although of course one or more of the materials used may be sterile and pyrogen-free anyway. Where all materials employed are sterile and pyrogen-free, there may then be no need for passing the resultant solution through a sterilising filter.

With the solutions of the invention it is possible to obtain compositions having a very high concentration of the anthracycline glycoside active substance even at 50 mg/ml. This constitutes a great advantage over the presently available lyophilized preparates wherein high concentrations of anthracycline glycoside can only be obtained with difficulty because of solubilization problems encountered in reconstitution, mainly with saline. The presence of the excipient, e.g. lactose, in the lyophilized cake, and its generally high proportion in respect of the active substance, even up to 5 parts of excipient per part of active substance, has a negative effect on solubilization so that difficulties may arise in obtaining dissolution of the lyophilized cake, especially for concentrations of anthracycline glycoside higher than 2 mg/ml.

The solutions of the invention are characterized by a good stability. Solutions in various solvents and with different pH's and concentrations have been found to be stable for long periods at temperatures accepted for the storage of pharmaceutical preparations. This is illustrated in the Examples which follow.

Owing to the well known anti-tumor activity of the anthracycline glycoside active drug substance, the pharmaceutical compositions of the invention are useful for treating tumors in both human and animal hosts. Examples of tumors that can be treated are, for instance, sarcomas, including osteogenic and soft tissue sarcomas, carcinomas, e.g., breast-, lung-, bladder-, thyroid-, prostate- and ovarian carcinoma, lymphomas, including Hodgkin and non-Hodgkin lymphomas, neuroblastoma, melanoma, myeloma, Wilms tumor, and leukemias, including acute lymphoblastic leukemia and acute myeloblastic leukemia.

Examples of specific tumours that can be treated are Moloney Sarcoma Virus, Sarcoma 180 Ascites, solid Sarcoma 180, gross transplantable leukemia, L 1210 leukemia and lymphocytic P 388 leukemia.

Inhibition of the growth of a tumour, in particular one of those indicated above, can be achieved by administering to a host suffering from a said tumour an injectable solution according to the invention containing the active drug substance in an amount sufficient to inhibit the growth of said tumour.

The injectable solutions of the invention are administered by rapid e.g. intravenous injection or infusion according to a variety of possible dose schedules.

A suitable dose schedule for doxorubicin may be, for example, of 60 to 75 mg of active drug substance per $m^2$ of body surface given as a single rapid infusion and repeated at 21 days. An alternative schedule may be of 30 $mg/m^2$ day be intravenous route for 3 days, every 28 days. Suitable dosages for 4'-epi-doxorubicin and 4'-desoxy-doxorubicin may be, for instance, of 75 to 90 $mg/m^2$ given in a single infusion to be repeated at 21 days, and similar dosages may be useful also for 4'-desoxy-4'-iodo-doxorubicin.

Idarubicin, i.e. 4-demethoxy-daunorubicin, may be, e.g. administered intravenously at a single dose of 13-15 $mg/m^2$ every 21 days in the treatment of solid tumours, while in the treatment of leukemias a preferred dose schedule is, e.g., of 10-12 $mg/m^2$ day by intravenous route for 3 days, to be repeated every 15-21 days. Similar dosages may be, e.g., followed for daunorubicin.

The following Examples illustrate the invention.

Example 1: Doxorubicin.HCl solutions in sterile water, 5% dextrose or 0.9% saline

Doxorubicin.HCl was dissolved at a concentration of 2 mg/ml in I = 0.05, pH 2.5 and 3.0 glycine buffers.

Each solution was filtered through a 0.22 $\mu$m microporous membrane under nitrogen pressure. 5.0 ml of each solution were stored at 55°C in glass vials of glass type I, 8ml top capacity vial, Teflon (Trade Mark)-faced chlorobutyl rubber bung, aluminium seal. Each solution was analysed at predetermined times (up to 120 hours) for doxorubicin.HCl assay and pH. The results are shown in Tables 1, 2 and 3 which give the doxorubicin.HCl residual concentration and percent stability at 55°C, at different pHs and times of storage for sterile water, 5% dextrose and 0.9% saline solutions, respectively.

The doxorubicin.HCl assays are the mean of three independent determinations performed by the US Pharmacopoeia (USP high performance liquid chromatography (HPLC) method (USP XXI)). At each pH value, the pseudo-first order rate constants ($K_{obs}$) for the degradation were calculated by linear regression analysis of the natural logarithm of the residual concentration of doxorubicin.HCl ($|Dx|_t$) versus time as depicted by the following equation:

$$\ln |Dx|_t = \ln |Dx|_o - K_{obs} \cdot t$$

Tables 4, 5 and 6 give the observed rate constants ($K_{obs}$) for the degradation kinetics of doxorubicin.HCl at 55°C and at different pHs for sterile water, 5% dextrose and 0.9% saline solutions, respectively.

Table 1- Accelerated (55°C) stability data of 2 mg/ml doxorubicin·HCl solutions in sterile water at various pHs

| Buffers | Tests | Time (hours) | | | | | | |
|---|---|---|---|---|---|---|---|---|
| | | 0 | 8 | 16 | 24 | 48 | 72 | 120 |
| pH 2.5 glycine-HCl | Doxorubicin·HCl assay . mg/ml | 1.992 | 1.926 | 1.835 | 1.718 | 1.557 | 1.00 | |
| | . % stability | 100.0 | 96.7 | 92.1 | 86.2 | 78.2 | 50.2 | |
| | pH | 2.51 | 2.50 | 2.50 | 2.52 | ·2.51 | 2.52 | |
| pH 3.0 glycine-HCl | Doxorubicin·HCl assay . mg/ml | 2.003 | 1.958 | 1.881 | 1.831 | 1.696 | 1.525 | 1.258 |
| | . % stability | 100.0 | 97.8 | 93.9 | 91.4 | 84.7 | 76.1 | 62.8 |
| | pH | 3.00 | 3.03 | 3.02 | 3.02 | 3.01 | 3.02 | 3.00 |

Table 2 - Accelerated (55°C) stability data of 2 mg/ml doxorubicln·HCl solutions In 5% dextrose at various pHs

| Buffers | Tests | Time (hours) | | | | | | | | |
|---------|-------|---|---|---|---|---|---|---|---|---|
| | | 0 | 8 | 16 | 24 | 34 | 48 | 72 | 96 | 120 |
| | Doxorubicin·HCl assay | | | | | | | | | |
| pH 2.5 | . mg/ml | 1.967 | 1.897 | 1.822 | 1.760 | 1.682 | 1.499 | 1.305 | | |
| glycine-HCl | . % stability | 100.0 | 96.4 | 92.6 | 89.5 | 85.5 | 76.2 | 66.3 | | |
| | pH | 2.56 | 2.56 | 2.56 | 2.58 | 2.60 | 2.56 | 2.61 | | |
| | Doxorubicin·HCl assay | | | | | | | | | |
| pH 3.0 | . mg/ml | 1.975 | | 1.908 | 1.832 | | 1.645 | 1.508 | 1.344 | 1.206 |
| glycine-HCl | . % stability | 100.0 | | 96.6 | 92.7 | | 83.3 | 76.4 | 68.0 | 61.1 |
| | pH | 3.04 | | 3.05 | 3.05 | | 3.06 | 3.00 | 3.13 | 3.10 |

EP 0 273 603 B1

EP 0 273 603 B1

Table 3 - Accelerated (55°C) stability data of 2 mg/ml doxorubicin·HCl solutions in 0.9% saline
at various pHs

| Buffers | Tests | Time (hours) | | | | | | | | | |
|---|---|---|---|---|---|---|---|---|---|---|---|
| | | 0 | 4 | 8 | 16 | 24 | 34 | 48 | 72 | 96 | 120 |
| | Doxorubicin·HCl assay | | | | | | | | | | |
| pH 2.5 | . mg/ml | 1.946 | | 1.875 | 1.670 | 1.602 | 1.368 | 1.132 | | | |
| glycine- | . % stability | 100.0 | | 96.3 | 85.8 | 82.3 | 70.3 | 58.1 | | | |
| HCl | pH | 2.59 | | 2.59 | 2.59 | 2.58 | 2.62 | 2.62 | | | |
| | Doxorubicin·HCl assay | | | | | | | | | | |
| pH 3.0 | . mg/ml | 1.994 | | | 1.818 | 1.771 | | 1.571 | 1.375 | 1.205 | 1.003 |
| glycine- | . % stability | 100.0 | | | 91.2 | 88.8 | | 78.8 | 69.0 | 60.4 | 50.3 |
| HCl | pH | 3.06 | | | 3.07 | 3.07 | | 3.08 | 3.13 | 3.14 | 3.12 |

Table 4

| $K_{obs}$ values (1/days) for the degradation of doxorubicin.HCl 2 mg/ml solutions in sterile water at various pHs at 55°C | | | |
|---|---|---|---|
| Buffer | pH | $K_{obs}$ x $10^3$ | 95% confidence limits |
| . Glycine-HCl (I = 0.05)) | 2.5 | 138.3 | ± 0.6 |
| . Glycine-HCl (I = 0.05) | 3.0 | 93.1 | ± 4.6 |

Table 5

| $K_{obs}$ values (1/days) for the degradation of doxorubicin.HCl 2 mg/ml solutions in 5% dextrose at various pHs at 55°C | | | |
|---|---|---|---|
| Buffer | pH | $K_{obs}$ x $10^3$ | 95% confidence limits |
| . Glycine-HCl (I = 0.05) | 2.5 | 138.7 | ± 9.9 |
| . Glycine-HCl (I = 0.05) | 3.0 | 100.5 | ± 5.9 |

Table 6

| $K_{obs}$ values (1/days) for the degradation of doxorubicin.HCl 2 mg/ml solutions in 0.9% saline at various pHs at 55°C | | | |
|---|---|---|---|
| Buffer | pH | $K_{obs}$ x $10^3$ | 95% confidence limis |
| . Glycine-HCl (I = 0.05) | 2.5 | 276.5 | ± 30.2 |
| . Glycine-HCl (I = 0.05) | 3.0 | 133.2 | ± 8.0 |

Example 2 : 4'-epi-doxorubicin (i.e. epirubicin) solutions

Solutions of epirubicin were prepared in the same fashion as the corresponding doxorubicin solutions of Example 1. They were then tested for stability in the same way. The results are presented in Tables 7 to 12.

Table 7 - Accelerated (55°C) stability data of 2 mg/ml epirubicin.HCl solutions in sterile water at various pHs

| Buffers | Tests | Time (hours) | | | | | | |
|---|---|---|---|---|---|---|---|---|
| | | 0 | 16 | 24 | 48 | 72 | 96 | 120 |
| pH 2.5 glycine-HCl | Epirubicin.HCl assay | | | | | | | |
| | . mg/ml | 2.033 | 1.972 | 1.923 | 1.823 | 1.777 | 1.622 | 1.572 |
| | . % initial | 100.0 | 99.9 | 94.6 | 89.7 | 87.4 | 79.8 | 77.3 |
| | pH | 2.5 | 2.4 | 2.5 | 2.5 | 2.4 | 2.4 | 2.4 |
| pH 3.0 glycine-HCl | Epirubicin.HCl assay | | | | | | | |
| | . mg/ml | 2.055 | 2.008 | 1.917 | 1.764 | 1.709 | 1.571 | 1.494 |
| | . % initial | 100.0 | 97.7 | 93.3 | 85.8 | 83.2 | 76.4 | 72.7 |
| | pH | 2.9 | 2.9 | 2.9 | 2.8 | 2.9 | 2.9 | 2.9 |

Table 8 - Accelerated (55°C) stability data of 2 mg/ml epirubicin.HCl solutions in 0.9% Sodium Chloride Injection at various pHs

| Buffers | Tests | Time (hours) | | | | | | | | | | |
|---|---|---|---|---|---|---|---|---|---|---|---|---|
| | | 0 | 4 | 8 | 16 | 24 | 36 | 48 | 72 | 96 | 120 | 144 |
| pH 2.5 glycine-HCl | Epirubicin.HCl assay | | | | | | | | | | | |
| | . mg/ml | 2.077 | | 2.066 | 1.987 | | | 1.781 | 1.665 | 1.449 | 1.285 | 1.175 |
| | . % initial | 100.0 | n.d. | 99.5 | 95.7 | n.d. | n.d. | 85.7 | 80.2 | 69.8 | 61.9 | 56.6 |
| | pH | 2.4 | | 2.4 | 2.4 | | | 2.4 | 2.4 | 2.4 | 2.4 | 2.4 |
| pH 3.0 glycine-HCl | Epirubicin.HCl assay | | | | | | | | | | | |
| | . mg/ml | 2.056 | | | 1.951 | 1.934 | 1.869 | 1.784 | 1.668 | 1.483 | 1.349 | 1.253 |
| | . % initial | 100.0 | n.d. | n.d. | 94.8 | 94.0 | 90.8 | 86.7 | 81.0 | 72.1 | 65.5 | 60.9 |
| | pH | 3.0 | | | 2.9 | 2.9 | 2.9 | 2.9 | 2.9 | 2.9 | 2.9 | 2.9 |

n.d. = not determined

Table 9 - Accelerated (55°C) stability data of 2 mg/ml epirubicin.HCl solutions in 5% Dextrose at various pHs

| Buffers | Tests | Time (hours) | | | | | | | | | | |
|---|---|---|---|---|---|---|---|---|---|---|---|---|
| | | 0 | 4 | 8 | 16 | 24 | 36 | 48 | 72 | 96 | 120 | 144 |
| pH 2.5 glycine-HCl | Epirubicin.HCl assay | | | | | | | | | | | |
| | . mg/ml | 2.105 | | | 1.921 | 1.909 | 1.815 | 1.819 | 1.624 | 1.521 | | 1.264 |
| | . % initial | 100.0 | n.d. | n.d. | 91.3 | 90.7 | 86.2 | 86.4 | 77.1 | 72.3 | n.d. | 60.0 |
| | pH | 2.4 | | | 2.3 | 2.3 | 2.3 | 2.3 | 2.3 | 2.3 | | 2.3 |
| pH 3.0 glycine-HCl | Epirubicin.HCl assay | | | | | | | | | | | |
| | . mg/ml | 2.029 | | 1.990 | 1.914 | 1.949 | 1.866 | 1.743 | | 1.562 | 1.442 | 1.318 |
| | . % initial | 100.0 | n.d. | 98.1 | 94.3 | 96.1 | 92.0 | 85.9 | n.d. | 77.0 | 71.1 | 65.0 |
| | pH | 2.9 | | 2.8 | 2.9 | 2.8 | 2.8 | 2.8 | | 2.8 | 2.8 | 2.8 |

n.d. = not determined

Table 10

| $K_{obs}$ values (1/days) for the degradation of epirubicin.HCl 2 mg/ml solutions in Water for Injection at various pHs at 55°C | | | |
|---|---|---|---|
| Buffer | pH | $K_{obs}$ x $10^3$ | 95% confidence limits |
| . Glycine-HCl (I = 0.05) | 2.5 | 55.1 | ± 4.0 |
| . Glycine-HCl (I = 0.05) | 3.0 | 66.8 | ± 5.4 |

Table 11

| $K_{obs}$ values (1/days) for the degradation of epirubicin.HCl 2 mg/ml solutions in 0.9% Sodium Chloride Injection at various pHs at 55°C | | | |
|---|---|---|---|
| Buffer | pH | $K_{obs}$ x $10^3$ | 95% confidence limits |
| . Glycine-HCl (I = 0.05) | 2.5 | 97.3 | ± 6.4 |
| . Glycine-HCl (I = 0.05) | 3.0 | 84.8 | ± 6.5 |

Table 12

| $K_{obs}$ values (1/days) for the degradation of epirubicin.HCl 2 mg/ml solutions in 5% Dextrose solution at various pHs at 55°C | | | |
|---|---|---|---|
| Buffer | pH | $K_{obs}$ x $10^3$ | 95% confidence limits |
| . Glycine-HCl (I = 0.05) | 2.5 | 81.1 | ± 6.6 |
| . Glycine-HCl (I = 0.05) | 3.0 | 70.9 | ± 4.8 |

**Claims**
**Claims for the following Contracting States : AT, BE, CH, DE, FR, GB, IL, LI, LU, NL, SE**

1. A ready-to-use, storage stable, sterile, pyrogen-free, injectable anthracycline glycoside solution which consists of a physiologically acceptable salt of an anthracycline glycoside dissolved in a physiologically acceptable aqueous solvent therefor at an anthracycline glycoside concentration of from 0.1 to 50 mg/ml, and which has not been reconstituted from a lyophilizate; characterised in that the pH of the solution has been adjusted to from 2.5 to 3.5 by means of a glycine buffer.

2. A solution according to claim 1 in a sealed container.

3. A solution according to claim 1 or 2 wherein the anthracycline glycoside is doxorubicin or 4'-epidoxorubicin.

4. A solution according to any one of the preceding claims having a pH of from 3 to 3.5.

5. A solution according to any one of claims 1 to 3 having a pH of about 3.

6. A solution according to any one of the preceding claims, wherein the said salt is the salt with hydrochloric acid.

7. A solution according to any one of the preceding claims which further contains one or more formulation adjuvants selected from a co-solubilising agent, a tonicity adjustment agent, a preservative and a pharmaceutically acceptable chelating agent.

8. A solution according to claim 7, containing dextrose, lactose, sorbitol or mannitol as a tonicity adjustment agent.

9. A solution according to any one of the preceding claims, wherein the physiologically acceptable solvent is water or physiological saline or an aqueous 5% dextrose solution.

10. A process for producing a ready-to-use, storage-stable, sterile, pyrogen-free, injectable anthracycline glycoside solution which process comprises dissolving a physiologically acceptable salt of an anthracycline glycoside, which salt is not in the form of a lyophilizate, in a physiologically acceptable aqueous solvent therefor at an anthracycline glycoside concentration of from 0.1 to 50 mg/ml and the process being effected in such a manner that the resultant solution is sterile and pyrogen-free; characterised in that a glycine buffer is added to adjust the pH of the solution to from 2.5 to 3.5 as desired.

11. A process according to claim 10 wherein the solution is passed through a sterilising filter after addition of the glycine buffer.

12. A process according to claim 10 or 11, wherein the resultant solution is introduced into a container which is then sealed.

13. A process according to any one of claims 10 to 12, wherein the anthracycline glycoside is doxorubicin or 4'-epi-doxorubicin.

14. A process according to any one of claims 10 to 13, wherein the pH is adjusted to from 3 to 3.5.

15. A process according to any one of claims 10 to 13, wherein the pH is adjusted to about 3.

16. A process according to any one of claims 10 to 15, wherein the said salt is the salt with hydrochloric acid.

17. A process according to any one of claims 10 to 16, which process comprises:
(i) dissolving the physiologically acceptable salt in the physiologically acceptable aqueous solvent;
(ii) adding one or more formulation adjuvants selected from co-solubilizing agents, tonicity adjustment agents, preservatives and pharmaceutically acceptable chelating agents; and
(iii) adding the glycine buffer.

18. A process according to claim 17, wherein dextrose, lactose, sorbitol or mannitol is added as a tonicity adjustment agent.

19. A process according to any one of claims 10 to 18, wherein the physiologically acceptable aqueous solvent is water or physiological saline or an aqueous 5% dextrose solution.

**Claims for the following Contracting States : ES, GR**

1. A process for producing a ready-to-use, storage-stable, sterile, pyrogen-free, injectable anthracycline glycoside solution, which process comprises dissolving a physiologically acceptable salt of an anthracycline glycoside, which salt is not in the form of a lyophilizate, in a physiologically acceptable aqueous solvent therefor at an anthracycline glycoside concentration of from 0.1 to 50 mg/ml and the process being effected in such a manner that the resultant solution is sterile and pyrogen-free; characterised in that a glycine buffer is added to adjust the pH of the solution to from 2.6 to 3.5 as desired.

2. A process according to claim 1 wherein the solution is passed through a sterilising filter after addition of the glycine buffer.

**3.** A process according to claim 1 or 2, wherein the resultant solution is introduced into a container which is then sealed.

**4.** A process according to any one of the preceding claims wherein the anthracycline glycoside is doxorubicin or 4'-epi-doxorubicin.

**5.** A process according to any one of the preceding claims wherein the pH is adjusted to from 3 to 3.5.

**6.** A process according to any one of claims 1 to 4, wherein the pH is adjusted to about 3.

**7.** A process according to any one of the preceding claims wherein the said salt is the salt with hydrochloric acid.

**8.** A process according to any one of the preceding claims, which process comprises:
(i) dissolving the physiologically acceptable salt in the physiologically acceptable aqueous solvent;
(ii) adding one or more formulation adjuvants selected from co-solubilizing agents, tonicity adjustment agents, preservatives and pharmaceutically acceptable chelating agents; and
(iii) adding the glycine buffer.

**9.** A process according to claim 8 wherein dextrose, lactose, sorbitol or mannitol is added as a tonicity adjustment agent.

**10.** A process according to any one of the preceding claims, wherein the physiologically acceptable aqueous solvent is water or physiological saline or an aqueous 5% dextrose solution.

**Revendications**
**Revendications pour les Etats contractants suivants : AT, BE, CH, DE, FR, GB, IL, LI, LU, NL, SE**

**1.** Une solution injectable d'anthracycline-glucoside apyrogène, stérile, stable au stockage, prête à l'emploi qui consiste en un sel physiologiquement acceptable d'un anthracycline-glucoside dissous dans un solvant aqueux physiologiquement acceptable à une concentration d'anthracycline-glucoside de 0,1 à 50 mg/ml et qui n'a pas été reconstituée à partir d'un lyophilisat ; caractérisée en ce que le pH de la solution a été réglé à 2,5-3,5 au moyen d'un tampon à la glycine.

**2.** Une solution selon la revendication 1, dans un récipient bouché hermétiquement.

**3.** Une solution selon la revendication 1 ou 2, dans laquelle l'anthracycline-glucoside est la doxorubicine ou la 4'-épi-doxorubicine.

**4.** Une solution selon l'une quelconque des revendications précédentes ayant un pH de 3 à 3,5.

**5.** Une solution selon l'une quelconque des revendications 1 à 3, ayant un pH d'environ 3.

**6.** Une solution selon l'une quelconque des revendications précédentes dans laquelle ledit sel est le sel de l'acide chlorhydrique.

**7.** Une solution selon l'une quelconque des revendications précédentes, qui contient en outre un ou plusieurs auxiliaires de formulation choisis parmi un agent cosolubilisant, un agent de réglage de la tonicité, un conservateur et un agent chélatant acceptable en pharmacie.

**8.** Une solution selon la revendication 7, contenant du dextrose, du lactose, du sorbitol ou du mannitol comme agent de réglage de la tonicité.

**9.** Une solution selon l'une quelconque des revendications précédentes dans laquelle le solvant physiologiquement acceptable est l'eau ou la solution salée physiologique ou une solution aqueuse de dextrose à 5%.

**10.** Un procédé pour produire une solution injectable d'anthracycline-glucoside apyrogène, stérile, stable au stockage, prête à l'emploi, procédé qui comprend la dissolution d'un set physiologiquement acceptable d'un anthracycline-glucoside, sel qui n'est pas sous la forme d'un lyophilisat, dans un solvant aqueux physiologiquement acceptable pour celui-ci à une concentration d'anthracycline-glucoside de 0,1 à 50 mg/ml et le procédé étant mis en oeuvre de telle manière que la solution résultante soit stérile et apyrogène ; caractérisé en ce que l'on ajoute un tampon à la glycine pour régler le pH de la solution à 2,5-3,5 comme on le désire.

11. Un procédé selon la revendication 10, dans lequel on fait passer la solution à travers un filtre stérilisant après addition du tampon à la glycine.

12. Un procédé seton la revendication 10 ou 11, dans lequel on introduit la solution résultante dans un récipient qui est ensuite bouché hermétiquement.

13. Un procédé selon l'une quelconque des revendications 10 à 12, dans lequel l'anthracycline-glucoside est la doxorubicine ou la 4'-épidoxorubicine.

14. Un procédé selon l'une quelconque des revendications 10 à 13, dans lequel le pH est réglé à 3-3,5.

15. Un procédé selon l'une quelconque des revendications 10 à 13, dans lequel le pH est réglé à environ 3.

16. Un procédé seton l'une quelconque des revendications 10 à 15, dans lequel ledit sel est le sel de l'acide chlorhydrique.

17. Un procédé seton l'une quelconque des revendications 10 à 16, procédé qui comprend les étapes suivantes :
(i) on dissout le sel physiologiquement acceptable dans le solvant aqueux physiologiquement acceptable ;
(ii) on ajoute un ou plusieurs auxiliaires de formulation choisis parmi les agents cosolubilisants, les agents de réglage de la tonicité, les conservateurs et les agents chélatants acceptables en pharmacie ; et
(iii) on ajoute le tampon à la glycine.

18. Un procédé selon la revendication 17, dans lequel on ajoute comme agent de réglage de la tonicité du dextrose, du lactose, du sorbitol ou du mannitol.

19. Un procédé selon l'une quelconque des revendications 10 à 18, dans lequel le solvant aqueux physiologiquement acceptable est l'eau ou la solution salée physiologique ou le dextrose aqueux à 5%.

**Revendications pour les Etats contractants suivants : ES, GR**

1. Un procédé pour produire une solution injectable d'anthracycline-glucoside apyrogène, stérile, stable au stockage, prête à l'emploi, procédé qui comprend la dissolution d'un sel physiologiquement acceptable d'un anthracycline-glucoside, sel qui n'est pas sous la forme d'un lyophilisat, dans un solvant aqueux physiologiquement acceptable pour celui-ci à une concentration d'anthracycline-glucoside de 0,1 à 50 mg/ml et le procédé étant mis en oeuvre de telle manière que la solution résultante soit stérile et apyrogène ; caractérisé en ce que l'on ajoute un tampon à la glycine pour régler le pH de la solution à 2,6-3,5 comme on le désire.

2. Un procédé selon la revendication 1, dans lequel on fait passer la solution à travers un filtre stérilisant après addition du tampon à la glycine.

3. Un procédé selon la revendication 1 ou 2, dans lequel on introduit la solution résultante dans un récipient qui est ensuite bouché hermétiquement.

4. Un procédé selon l'une quelconque des revendications précédentes, dans lequel l'anthracycline-glucoside est la doxorubicine ou la 4'-épidoxorubicine.

**5.** Un procédé selon l'une quelconque des revendications précédentes, dans lequel le pH est réglé à 3-3,5.

**6.** Un procédé selon l'une quelconque des revendications 1 à 4, dans lequel le pH est réglé à environ 3.

**7.** Un procédé selon l'une quelconque des revendications précédentes, dans lequel ledit sel est le sel de l'acide chlorhydrique.

**8.** Un procédé selon l'une quelconque des revendications précédentes, procédé qui comprend les étapes suivantes :
(i) on dissout le sel physiologiquement acceptable dans le solvant aqueux physiologiquement acceptable ;
(ii) on ajoute un ou plusieurs auxiliaires de formulation choisis parmi les agents cosolubilisants, les agents de réglage de la tonicité, les conservateurs et les agents chélatants acceptables en pharmacie ; et
(iii) on ajoute le tampon à la glycine.

**9.** Procédé selon la revendication 8, dans lequel on ajoute comme agent de réglage de la tonicité du dextrose, du lactose, du sorbitol ou du mannitol.

**10.** Un procédé selon l'une quelconque des revendications précédentes, dans lequel le solvant aqueux physiologiquement acceptable est l'eau ou la solution salée physiologique ou le dextrose aqueux à 5%.

**Patentansprüche**

**Patentansprüche für folgende Vertragsstaaten : AT, BE, CH, DE, FR, GB, IT, LI, LU, NL, SE**

**1.** Fertig brauchbare, lagerstabile, sterile, pyrogenfreie, injizierbare Anthracyclinglycosidlösung, bestehend aus einem physiologisch akzeptablen Salz aus einem Anthracyclinglycosid, das in einem physiologisch akzeptablen wässrigen Lösungsmittel dafür bei einer Anthracyclinglycosidkonzentration von 0,1 bis 50 mg/ml aufgelöst ist, und die nicht aus einem Lyophilisat wieder hergestellt ist;
dadurch **gekennzeichnet,** daß
der pH-Wert der Lösung auf 2.5 bis 3.5 mit Hilfe eines Glycinpuffers eingestellt ist.

**2.** Lösung nach Anspruch 1, in einem geschlosenen Behälter.

**3.** Lösung nach Anspruch 1 oder 2,
dadurch **gekennzeichnet,** daß
das Anthracyclinglycosid Doxorubicin oder 4'-Epidoxorubicin ist.

**4.** Lösung nach einem der vorhergehenden Ansprüche mit einem pH-Wert von 3 bis 3.5.

**5.** Lösung nach einem der Ansprüche 1 bis 3 mit einem pH-Wert von etwa 3.

**6.** Lösung nach einem der vorhergehenden Ansprüche,
dadurch **gekennzeichnet,** daß
das Salz das Salz mit Salzsäure ist.

**7.** Lösung nach einem der vorhergehenden Ansprüche,
dadurch **gekennzeichnet,** daß
sie weiterhin einen oder mehrere Formulierungshilfsmittel umfaßt, ausgewählt aus einem Co-Lösungsmittel, einem Tonizitätseinstellmittel, einem Konservierungsmittel und einem pharmazeutisch akzeptablen Chelatisierungsmittel.

**8.** Lösung nach Anspruch 7,
dadurch **gekennzeichnet,** daß
sie Dextrose, Lactose, Sorbit oder Mannit als ein Tonizitätseinstellmittel enthällt.

**9.** Lösung nach einem der vorhergehenden Ansprüche,

dadurch **gekennzeichnet,** daß
das physiologisch akzeptable Lösungsmittel Wasser oder physiologische Kochsalzlösung oder eine wässrige 5%ige Dextroselösung ist.

10. Verfahren zur Herstellung einer fertig brauchbaren, lagerstabilen, sterilen, pyrogen-freien, injizierbaren Anthracyclinglycosidlösung, wobei das Verfahren das Auflösen eines physiologisch akzeptablen Salzes eines Anthracyclinglycosids, wobei das Salz nicht in der Form eines Lyophilisats vorliegt, in einem physiologisch akzeptablen wässrigen Lösungsmittel dafür bei einer Anthracyclinglycosidkonzentration von 0,1 bis 50 mg/ml umfaßt und wobei das Verfahren in einer solchen Weise durchgeführt wird, daß die resultierende Lösung steril und pyrogenfrei ist,
dadurch **gekennzeichnet,** daß
ein Glycinpuffer zum Einstellen des pH-Wertes der Lösung auf 2.5 bis 3.5, wie gewünscht, zugegeben wird.

11. Verfahren nach Anspruch 10,
dadurch **gekennzeichnet,** daß
die Lösung durch ein Sterilisationsfilter nach der Zugabe des Glycinpuffers geleitet wird.

12. Verfahren nach Anspruch 10 oder 11,
dadurch **gekennzeichnet,** daß
die resultierende Lösung in einen Behälter gegeben wird, der dann abgedichtet wird.

13. Verfahren nach einem der Ansprüche 10 bis 12,
dadurch **gekennzeichnet,** daß
das Anthracyclinglycosid Doxorubicin oder 4'-Epi-doxorubicin ist.

14. Verfahren nach einem der Ansprüche 10 bis 13,
dadurch **gekennzeichnet,** daß
der pH-Wert auf 3 bis 3,5 eingestellt wird.

15. Verfahren nach einem der Ansprüche 10 bis 13,
dadurch **gekennzeichnet,** daß
der pH-Wert auf etwa 3 eingestellt wird.

16. Verfahren nach einem der Ansprüche 10 bis 15,
dadurch **gekennzeichnet,** daß
das Salz das Salz mit Salzsäure ist.

17. Verfahren nach einem der Ansprüche 10 bis 16,
**gekennzeichnet** durch:
(i) Auflösen des physiologisch akzeptablen Salzes in dem physiologisch akzeptablen wässrigen Lösungsmittel;
(ii) Zugabe von einem oder mehreren Formulierungshilfsmitteln, ausgewählt aus Colösungsmitteln, Tonizitätseinstellmitteln, Konservierungsmitteln und pharmazeutisch akzeptablen Chelatisierungsmitteln; und
(iii) Zugabe des Glycinpuffers.

18. Verfahren nach Anspruch 17,
dadurch **gekennzeichnet,** daß
Dextrose, Lactose, Sorbit oder Mannit als ein Tonizitätseinstellmittel verwendet wird.

19. Verfahren nach einem der Ansprüche 10 bis 18,
dadurch **gekennzeichnet,** daß
das physiologisch akzeptable wässrige Lösungsmittel Wasser oder physiologische Kochsalzlösung oder eine wässrige 5%ige Dextroselösung ist.

**Patentansprüche für folgende Vertragsstaaten : ES, GR**

**EP 0 273 603 B1**

1. Verfahren zur Herstellung einer fertig brauchbaren, lagerstabilen, sterilen, pyrogenfreien, injizierbaren Anthracyclinglycosidlösung, wobei das Verfahren das Auflösen eines physiologisch akzeptablen Salzes eines Anthracyclinglycosids, wobei das Salz nicht in der Form eines Lyophilisats vorliegt, in einem physiologisch akzeptablen wässrigen Lösungsmittel dafür bei einer Anthracyclinglycosidkonzentration von 0,1 bis 50 mg/ml umfaßt und wobei das Verfahren auf eine solche Weise durchgeführt wird, daß die resultierende Lösung steril und pyrogenfrei ist, dadurch **gekennzeichnet,** daß ein Glyzinpuffer zugegeben wird, um den pH-Wert der Lösung auf 2.6 bis 3.5, wie gewünscht,einzustellen.

2. Verfahren nach Anspruch 1, dadurch **gekennzeichnet,** daß die Lösung durch einen Sterilisationsfilter nach der Zugabe des Glycinpuffers geleitet wird.

3. Verfahren nach Anspruch 1 oder 2, dadurch **gekennzeichnet,** daß die resultierende Lösung in einen Behälter gegeben wird, der dann abgedichtet wird.

4. Verfahren nach einem der vorhergehenden Ansprüche, dadurch **gekennzeichnet,** daß das Anthracyclinglycosid Doxorubicin oder 4'-Epi-doxorubicin ist.

5. Verfahren nach einem der vorhergehenden Ansprüche, dadurch **gekennzeichnet,** daß der pH-Wert auf 3 bis 3,5 eingestellt wird.

6. Verfahren nach einem der Ansprüche 1 bis 4, dadurch **gekennzeichnet,** daß der pH-Wert auf etwa 3 eingestellt wird.

7. Verfahren nach einem der vorhergehenden Ansprüche, dadurch **gekennzeichnet,** daß das Salz das Salz mit Salzsäure ist.

8. Verfahren nach einem der vorhergehenden Ansprüche, **gekennzeichnet** durch:
   (i) Auflösen des physiologisch akzeptablen Salzes in dem physiologisch akzeptablen wässrigen Lösungsmittel;
   (ii) Zugabe von einem oder mehreren Formulierungshilfsmitteln, ausgewählt aus Colösungsmitteln, Tonizitätseinstellmitteln, Konservierungsmitteln und pharmazeutisch akzeptablen Chelatisierungsmitteln; und
   (iii) Zugabe des Glycinpuffers.

9. Verfahren nach Anspruch 8, dadurch **gekennzeichnet,** daß Dextrose, Lactose, Sorbit oder Mannit als ein Tonizitätseinstellmittel verwendet wird.

10. Verfahren nach einem der vorhergehenden Ansprüche, dadurch **gekennzeichnet,** daß das physiologisch akzeptable wässrige Lösungsmittel Wasser oder physiologische Kochsalzlösung oder eine wässrige 5%ige Dextroselösung ist.

16